# FASCICULE DE BREVET EUROPEEN

(11) **EP 0 406 129 B1**
(45) Date de publication et mention de la délivrance du brevet: **23.11.1994**
(21) Numéro de dépôt: 90420281.9
(22) Date de dépôt: 13.06.1990
(51) Int. Cl.: C07C 271/28, C07C 269/06

(54) **Procédé de préparation de méthylène di(phényluréthane)**
Verfahren zur Herstellung von Methylendi(phenylurethan)
Method for the preparation of methylene di(phenylurethane)

(30) Priorité: 29.06.1989 FR 8909002
(43) Date de publication de la demande: 02.01.1991
(73) Titulaire: RHONE-POULENC CHIMIE, 92408 Courbevoie Cédex (FR)
(72) Inventeur: Gubelmann, Michel, F-69002 Lyon (FR); Rochin, Christophe, F-69006 Lyon (FR); Allandrieu, Christian, F-69100 Villeurbanne (FR)
(74) Mandataire: Dubruc, Philippe

(56) Documents cités:
- EP-A- 0 110 732
- GB-A- 2 004 866
- GB-A- 2 054 584

## Description

La présente invention concerne un procédé de préparation de méthylènedi(phényluréthane). Elle a plus particulièrement trait à la préparation de méthylènedi(phényluréthane) par réarrangement de bis (N-carboalcoxyanilino) méthanes.

Le méthylènedi(phényluréthane) couramment appelé MDU est un intermédiaire utile à la fabrication du méthylènedi(phénylisocyanate) connu sous l'appelation MDI. En effet, le MDU peut être pyrolysé en MDI de manière en Soi connue. Le MDI est utile notamment en tant que matière première pour les mousses et élastomères de polyuréthanes.

Le MDI est fabriqué conventionnellement par phosgénation de la diamine qui résulte de la condensation de l'aniline et du formaldéhyde. Le produit commercial est un mélange des divers isomères du MDI et d'oligomères couramment désignés par poly-méthylènedi(phénylisocyanate) PMDI, dont le MDI pur est susceptible d'être isolé.

Pour des raisons évidentes liées à la toxicité du phosgène et aux inconvénients associés à la génération d'acide chlorhydrique lors de l'étape de phosgénation, de nombreuses recherches ont été entreprises aux fins de proposer des voies d'accès au MDI ne nécessitant pas d'étape de phosgénation.

C'est ainsi qu'il a été proposé divers procédés de préparation du MDI à partir de N-phénylcarbamates d'alkyle comprenant une première étape de condensation du N-phénylcarbamate avec du formaldéhyde pour former un mélange renfermant du diphénylméthanedicarbamate et des poly-méthylènedi(phénylcarbamate), homologues supérieurs des méthylènedi(phénylcarbamate) (ou MDU), suivie d'une étape de décomposition thermique.

L'un des inconvénients présenté par ce type de procédés réside dans le fait que la proportion de MDI dinucléaires et, en particulier, de l'isomère 4,4' est insuffisante.

Un autre inconvénient présenté par ce type de procédés réside dans le fait que lors de l'étape de condensation il se forme aux côtés du diphénylméthanedicarbamate souhaité des proportions notables de composés tels les N-carboalcoxyanilinophénylméthanes, les bis(N-carboalcoxyanilino)méthanes, et les N,N'-dicarboalcoxyaminobenzylanilines et leurs dérivés de condensation supérieure.

Ces diverses impuretés sont gênantes pour la conversion du mélange réactionnel en diisocyanates recherchés.

Dans le brevet américain n° 4,146,727, il a été proposé de réarranger des impuretés de type N-benzylique de formule (I) 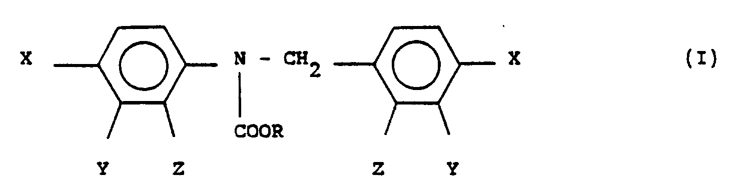
dans laquelle X, Y ou Z peuvent notamment représenter un groupe - NHCOOR, R est un groupe alkyle comportant de 1 à 3 atomes de carbone, leurs dimères, trimères, tétramères, etc, en diphénylméthanedicarbamate par leur mise en contact à une température comprise entre 50 et 170°C, de préférence entre 80 et 130°C avec une quantité catalytique d'un milieu acide protonique fort.

Il résulte de l'enseignement de ce document qu'essentiellement, seules des impuretés de type N-benzylique seraient formées et valorisables au moins partiellement par le procédé proposé.

Dans la demande de brevet français n° 2 460 972 (correspondant au brevet américain n° 4 319 018) il est proposé de réaliser l'étape de condensation du N-phénylcarbamate d'alkyle et du formaldéhyde ou d'une substance générant du formaldéhyde en présence simultanée d'au moins un composé qui peut notamment être choisi parmi les bis(N-carboalcoxyanilino) méthanes et les N,N'-dicarboalcoxyaminobenzylanilines et d'une solution aqueuse acide dont la concentration est ajustée de telle sorte que la cinétique de réaction soit acceptable et que les réactions secondaires soient maintenues à un niveau minimal, à une température comprise entre 10 et 150°C et de préférence entre 20 et 120°C.

Le procédé décrit dans cette demande ne permet pas d'éliminer totalement les impuretés en cause.

La Demanderesse dans le cadre de ses recherches a pu mettre en évidence que les N,N'-dicarboalcoxyaminobenzylanilines de formule 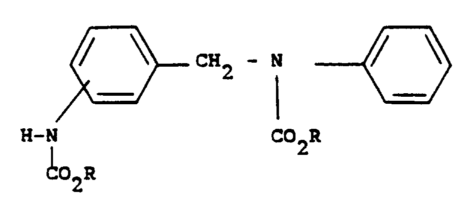
dans laquelle R représente un radical alkyle correspondant au reste alkyle du N-phénylcarbamate d'alkyle engagé dans la réaction de condensation avec un agent méthylénant, (composés à liaison méthylèneamino) sont non seulement des impuretés difficilement séparables mais aussi difficilement réarrangeables en produit recherché, Y compris en présence d'un N-phénylcarbamate d'alkyle. La Demanderesse sans vouloir être liée par une explication théorique, est conduite à penser que la formation de ces impuretés indésirables résulterait en quelque sorte du réarrangement d'un bis(N-carboalcoxyanilino) méthane répondant à la formule (I) 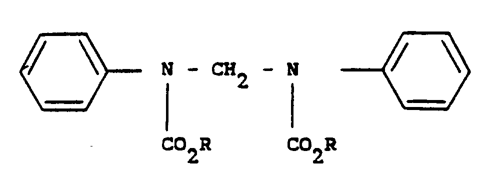
dans laquelle R représente un radical alkyle, dans les conditions jusqu'alors mises en oeuvre lors de l'étape de condensation et/ou de réarrangement.

Il a maintenant été trouvé qu'il est possible de réarranger les bis(N-carboalcoxyanilino) méthanes de formule (I) ci-avant en méthylène di(phényluréthane) avec une sélectivité améliorée et, de procéder audit réarrangement avec une sélectivité remarquable en isomère 4,4' dudit méthylènedi(phényluréthane).

La présente invention a donc pour objet un procédé de préparation de méthylènedi(phényluréthane) par réarrangement d'au moins un bis(N-carboalcoxyanilino) méthane, le cas échéant en présence d'au moins un N-phénylcarbarmate d'alkyle caractérisé en ce que le réarrangement est réalisé en présence d'acide fluorhydrique.

Les bis(N-carboalcoxyanilino) méthanes utilisables dans le cadre de la présente invention répondent à la formule (I) donnée ci-avant dans laquelle R représente un radical alkyle, ou cycloalkyle comportant de 1 à 6 et de préférence de 2 à 4 atomes de carbone.

Ces composés peuvent être produits par réaction de N-phénylcarbamate d' alkyle dans le chlorure de méthylène, servant à la fois de solvant de réaction et d'agent méthylénant en présence de soude à 50 % et d'un agent de tranfert de phase - cf. Annales de Quimica, ser. C, 80, 255 (1984).

Le réarrangement en cause peut être mis en oeuvre en présence d'un N-phénylcarbamate d'alkyle dont le reste alkyle comporte de 1 à 6 atomes de carbone et est avantageusement choisi identique aux restes alkyles des groupes carboalcoxy du composé de formule (I) mis en oeuvre.

Lorsqu'un tel carbamate est engagé dans le milieu réactionnel, le rapport molaire de celui-ci au bis(carboalcoxyanilino)méthane, qui peut varier dans de larges limites, sera avantageusement compris entre 0,5 et 10 et, de préférence entre 1 et 5.

Le réarrangement en cause est réalisé en présence d'acide fluorhydrique et, de préférence, en phase liquide. Cet acide est de préférence anhydre bien qu'un acide fluorhydrique contenant de l'eau ne soit pas à exclure. L'eau ralentit seulement la réaction. Le réarrangement en cause ne coproduisent pas d'eau, l'utilisation d'acide fluorhydrique anhydre apportera un avantage supplémentaire, à savoir, la possibilité de recycler aisément ledit acide.

La quantité d'acide fluorhydrique à mettre en oeuvre n'est pas critique. L'acide fluorhydrique peut être engagé en quantité importante par rapport aux réactifs de telle sorte qu'il constitue le solvant dans le milieu réactionnel. L'acide fluorhydrique peut être présent en quantité plus faible.

Pour une bonne mise en oeuvre de l'invention le rapport molaire de l'acide fluorhydrique au bis(N-carboalcoxyanilino) méthane est au moins égal à 10 et, de préférence, inférieur ou égal à 200. Selon une variante avantageuse ce rapport sera compris entre 25 et 100.

La température de réaction est en générale comprise entre - 20° et 80°C.

Pour une bonne mise en oeuvre du présent procédé la température sera comprise entre 0 et 60°C. En effet, au delà de 60°C on observe à la fois une diminution de la proportion d'isomère 4,4' du méthylènedi(phényluréthane), une augmentation en dérivés porteurs de 3 noyaux aromatiques, et une isomérisation de l'isomère 4,4' en isomère 2,4'.

On observe également, en dessous de 60°C, que la proportion de dérivés porteurs de 3 noyaux aromatiques reste faible dans le mélange réactionnel et que plus la température est basse plus la proportion d'isomère 4,4' recherché est importante dans le méthylànedi(phényluréthane) produit.

La pression n'est pas un paramètre essentiel du procédé. Toutefois, lorsque la température de réaction dépasse 20°C il est préférable d'opérer sous une pression supérieure à la pression atmosphérique pour maintenir l' acide fluorhydrique sous forme liquide.

Le procédé selon l'invention peut être mis en oeuvre dans l'acide fluorhydrique comme solvant ou dans un mélange d'acide fluorhydrique et d'un solvant organique. A titre d'exemples de solvants organiques utilisables dans le cadre du présent procédé on peut citer : des hydrocarbures aliphatiques tels l'hexane et l'heptane ; des hydrocarbures alicycliques tels le cyclohexane et le méthylcyclohexane ; et les hydrocarbures halogénés tels le chloroforme, le chlorure de méthylène, le chlorure d'éthylène, le chlorocyclohexane, le perchlorocyclohexane, le chlorobenzène et le dichlorobenzène. Quand on utilise un tel solvant, il représente au maximum 300 % et, de préférence, de 10 à 150 % en poids par rapport au bis(N-carboalcoxyanilino)méthane engagé.

La durée de réaction peut varier dans de larges limites ; elle est généralement comprise entre 15 mn et 8 heures.

La réaction peut être mise en oeuvre on discontinu ou en continu.

En fin de réaction ou du temps imparti à celle-ci on récupère le produit recherché par tous moyens appropriés, par exemple par évaporation de l'acide fluorhydrique.

Le procédé selon l'invention convient particulièrement bien au réarrangement du bis N-(carboéthoxyanilino) méthane, le cas échéant, en présence de N-phénylcarbamate d'éthyle.

Les exemples ci-après illustrent la présente invention.

Les conventions suivantes y sont utilisées :
- - MDU RR(%): : représente le rendement en méthylène di(carbanilate d'éthyle) calculé par rapport au nombre de mole initiales de groupes -CH₂-.
- - 3-Ph(%): : représente le rendement (RR) en composés présentant 3 noyaux aromatiques calculé de manière analogue
- - 4,4': : représente le méthylène-4,4' di(carbanilate d'éthyle) de formule : 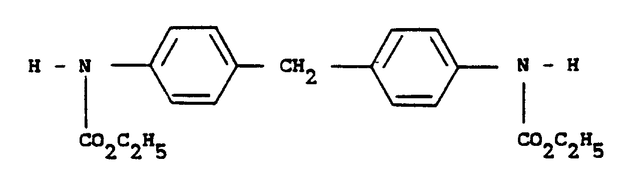
- - 2,4': : représente le méthylène-2,2' di(carbanilate d'éthyle)

. A représente le bis(N-carboéthoxyanili o méthane de formule

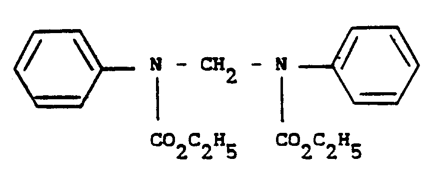

### EXEMPLE 1 à 3 :

Dans un réacteur en Hastelloy de 50 cm3 de capacité, muni d'une agitation magnétique, on charge :
10 mmol de bis(carboéthoxyanilino)méthane (A)
20 g (1 mol) d'acide fluorhydrique anhydre. Après deux heures de réaction à la température (T) on analyse le mélange par chromatographies en phase gazeuse et en phase liquide.

Les conditions particulières ainsi que les résultats obtenus figurent dans le tableau (I) ci-après :

Le taux de tranformation de A est de 100 % dans ces exemples :

**TABLEAU I**

| Ex. N° | T °C | MDU RR(%) | Répartition isomérique (%) | | 3-Ph (%) |
|---|---|---|---|---|---|
| | | | - 4,4' | - 2,4' | |
| 1 | 40 | 54 | 94 | 6 | 9 |
| 2 | 25 | 58 | 93 | 7 | 10 |
| 3 * | 40 | 91 | 93 | 7 | 3 |

| | | | | | |
|---|---|---|---|---|---|
| * dans cet exemple on a ajouté 30 mmol de phénylcarbamate d'éthyle à la charge. | | | | | |

Dans ces exemples on ne détecte pas la présence de composés à liaison méthylèneamino.

### EXAMPLES 4 ET 5 :

Dans un réacteur en Hastelloy de 50 cm3 de capacité, muni d'une agitation magnétique, on charge :
30 mmol de phénylcarbamate d'éthyle
10 mmol de bis(carboéthoxyanilino)méthane (A)
x cm3 de dichlorure de méthylène et (20-x)cm3 d'acide fluorhydrique anhydre.

En fin d'essais on analyse le mélange par chromatographies en phase gazeuse et en phase phase liquide.

Les conditions particulière ainsi que les résultats obtenus en 2 heures de réaction à 40°C sont rassemblés dans le tableau (II) ci-après :

**TABLEAU II**

| Ex. N° | HF cm3 | CH₂Cl₂ cm3 | MDU RR (%) | Répartition isomérique | | 3-Ph (%) |
|---|---|---|---|---|---|---|
| | | | | - 4,4' | - 2,4' | |
| 3 | 20 | 0 | 91 | 93 | 7 | 3 |
| 4 | 10 | 10 | ∼ 100 | 94 | 6 | ∼ 1 |
| 5 | 5 | 15 | 97 | 93 | 7 | 3 |

Dans ces exemples on ne détecte pas la présence de composés à liaison méthylèneamino.

Le taux de transformation de A est de 100 % dans ces exemples.

## Revendications

1. Procédé de préparation de méthylènedi(phényluréthane) par réarrangement d'au moins un bis(N-carboalcoxyanilino) méthane, le cas échéant en présence d'au moins un N-phénylcarbarmate d'alkyle caractérisé en ce que le réarrangement est réalisé en présence d'acide fluorhydrique.

2. Procédé selon la revendication 1, caractérisé en ce que le réarrangement est conduit en phase liquide.

3. Procédé selon la revendication 1 ou 2, caractérisé en ce qu'on utilise l'acide fluorhydrique anhydre.

4. Procédé selon l'une quelconque des revendications précédentes, caractérisé en ce que le rapport molaire du N-phénylcarbamate d'alkyle au bis(N-carboalcoxyanilino)méthane est compris entre 0,5 et 10 et, de préférence, entre 1 et 5.

5. Procédé selon l'une quelconque des revendications précédentes, caractérisé en ce que le bis (N-carboalcoxyanilino)méthane est le bis(N-carboéthoxyanilino)méthane.

6. Procédé selon l'une quelconque des revendications précédentes, caractérisé en ce que le réarrangement est conduit en présence d'un solvant organique.

7. Procédé selon la revendication 6, caractérisé en ce que le solvant organique est le chlorure de méthylène.

8. Procédé selon l'une quelconque des revendications précédentes, caractérisé en ce que le rapport molaire de l'acide fluorhydrique au bis(N-carboalcoxyanilino)méthane est supérieur ou égal à 10.

9. Procédé selon l'une quelconque des revendications précédentes, caractérisé en ce que le rapport molaire de l'acide fluorhydrique au bis(N-carboalcoxyanilino)méthane est inférieur ou égal à 200.

10. Procédé selon l'une quelconque des revendications précédentes, caractérisé en ce que le rapport molaire de l'acide fluorhydrique au bis(N-carboalcoxyanilino)méthane est compris entre 25 et 100.

11. Procédé selon l'une quelconque des revendications, caractérisé en ce que la température du réarrangement est comprise entre -20° et 80°C et, de préférence, entre 0 et 60°C.

## Claims

1. Process for the preparation of methylenedi(phenylurethane) by rearrangement of at least one bis(N-carbalkoxyanilino)methane, if appropriate in the presence of at least one alkyl N-phenylcarbamate, characterized in that the rearrangement is carried out in the presence of hydrofluoric acid.

2. Process according to Claim 1, characterized in that the rearrangement is conducted in liquid phase.

3. Process according to Claim 1 or 2, characterized in that anhydrous hydrofluoric acid is employed.

4. Process according to any one of the preceding claims, characterized in that the molar ratio of the alkyl N-phenylcarbamate to the bis(N-carbalkoxyanilino)methane is between 0.5 and 10 and, preferably, between 1 and 5.

5. Process according to any one of the preceding claims, characterized in that the bis(N-carbalkoxyanilino)methane is bis(N-carbethoxyanilino)methane.

6. Process according to any one of the preceding claims, characterized in that the rearrangement is conducted in the presence of an organic solvent.

7. Process according to Claim 6, characterized in that the organic solvent is methylene chloride.

8. Process according to any one of the preceding claims, characterized in that the molar ratio of hydrofluoric acid to the bis(N-carbalkoxyanilino)methane is higher than or equal to 10.

9. Process according to any one of the preceding claims, characterized in that the molar ratio of hydrofluoric acid to the bis(N-carbalkoxyanilino)methane is lower than or equal to 200.

10. Process according to any one of the preceding claims, characterized in that the molar ratio of hydrofluoric acid to the bis(N-carbalkoxyanilino)methane is between 25 and 100.

11. Process according to any one of the preceding claims, characterized in that the temperature of the rearrangement is between -20° and 80°C and, preferably, between 0 and 60°C.

## Patentansprüche

1. Verfahren zur Herstellung von Methylen(diphenylurethan) durch Umlagerung von mindestens einem Bis(N-carboalkoxyanilino)methan, gegebenenfalls in Anwesenheit von mindestens einem Alkyl-N-phenylcarbamat,
dadurch gekennzeichnet,
daß die Umlagerung in Anwesenheit von Fluorwasserstoffsäure ausgeführt wird.

2. Verfahren nach Anspruch 1,
dadurch gekennzeichnet,
daß die Umlagerung in flüssiger Phase ausgeführt wird.

3. Verfahren nach Anspruch 1 oder 2,
dadurch gekennzeichnet,
daß wasserfreie Fluorwasserstoffsäure verwendet wird.

4. Verfahren nach irgendeinem der vorangegangenen Ansprüche,
dadurch gekennzeichnet,
daß das molare Verhältnis von Alkyl-N-phenylcarbamat zu Bis(N-carboalkoxyanilino)methan zwischen 0,5 und 10, und vorzugsweise zwischen 1 und 5 liegt.

5. Verfahren nach irgendeinem der vorangegangenen Ansprüche,
dadurch gekennzeichnet,
daß das Bis(N-carboalkoxyanilino)methan Bis(N-carboethoxyanilino)methan ist.

6. Verfahren nach irgendeinem der vorangegangenen Ansprüche,
dadurch gekennzeichnet,
daß die Umlagerung in Gegenwart eines organischen Lösemittels ausgeführt wird.

7. Verfahren nach Anspruch 6,
dadurch gekennzeichnet,
daß das organische Lösemittel Methylenchlorid ist.

8. Verfahren nach irgendeinem der vorangegangenen Ansprüche,
dadurch gekennzeichnet,
daß das molare Verhältnis von Fluorwasserstoffsäure zu Bis(N-carboalkoxyanilino)methan größer als oder gleich 10 ist.

9. Verfahren nach irgendeinem der vorangegangenen Ansprüche,
dadurch gekennzeichnet,
daß das molare Verhältnis von Fluorwasserstoffsäure zu Bis(N-carboalkoxyanilino)methan geringer als oder gleich 200 ist.

10. Verfahren nach irgendeinem der vorangegangenen Ansprüche,
dadurch gekennzeichnet,
daß das molare Verhältnis von Fluorwasserstoffsäure zu Bis(N-carboalkoxyanilino)methan zwischen 25 und 100 liegt.

11. Verfahren nach irgendeinem der vorangegangenen Ansprüche,
dadurch gekennzeichnet,
daß die Temperatur der Umlagerung zwischen -20°C und 80°C, und vorzugsweise zwischen 0 und 60°C liegt.
